(19) Europäisches Patentamt
European Patent Office
Office européen des brevets

(11) Veröffentlichungsnummer: **0 301 449 B1**

# (12) EUROPÄISCHE PATENTSCHRIFT

(45) Veröffentlichungstag der Patentschrift: **13.05.92**

(51) Int. Cl.[5]: **A61K 31/135**

(21) Anmeldenummer: **88111911.9**

(22) Anmeldetag: **23.07.88**

## (54) Enantiomere des Propafenons enthaltende therapeutische Mittel.

(30) Priorität: **30.07.87 DE 3725273**

(43) Veröffentlichungstag der Anmeldung:
**01.02.89 Patentblatt 89/05**

(45) Bekanntmachung des Hinweises auf die Patenterteilung:
**13.05.92 Patentblatt 92/20**

(84) Benannte Vertragsstaaten:
**AT BE CH DE ES FR GB GR IT LI NL SE**

(56) Entgegenhaltungen:

**LIEBIGS ANNALEN DER CHEMIE, Nr. 6, Juni 1987, Seiten 561-563, VHC Verlagsgesellschaft mbH, Weinheim, DE; G. BLASCHKE et al.: "Racemattrennung von Propafenon und Diprafenon, Konfiguration der Propafenon-Enantiomeren"**

**IDEM**

(73) Patentinhaber: **BASF Aktiengesellschaft**
**Carl-Bosch-Strasse 38**
**W-6700 Ludwigshafen(DE)**

(72) Erfinder: **Franke, Albrecht, Dr.**
**Mandelring 11**
**W-6706 Wachenheim(DE)**
Erfinder: **Schlecker, Rainer, Dr.**
**Suedring 8**
**W-6719 Bissersheim(DE)**
Erfinder: **Gries, Josef, Dr.**
**Roemerweg 43**
**W-6706 Wachenheim(DE)**
Erfinder: **Von Philipsborn, Gerda, Dr.**
**Naechstenbacher Weg 35**
**W-6940 Weinheim(DE)**
Erfinder: **Unger, Liliane, Dr.**
**Waltraudenstrasse 14**
**W-6700 Ludwigshafen(DE)**

EP 0 301 449 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**THE JOURNAL OF PHARMACOLOGY AND EXPERIMENTAL THERAPEUTICS, Band 228, Nr. 2, Februar 1984, Seiten 461-466, The American Society for Pharmacology and Experimental Therapeutics, US; A.A. McLEOD et al.: "Demonstration of beta adrenoceptor blokkade by propafenone hydrochloride: clinical pharmacologic, radioligand binding and adenylate cyclase activation studies"**

**EUROPEAN HEART JOURNAL, Band 8, Nr. 1, Januar 1987, Seiten 53-56, The European Society of Cardiology; E.C. CHERIEX et al.: "Lack of clinically significant beta-blocking effect of propafenone"**

**ARZNEIM.-FORSCH. (DRUG RES.), Band 26, Nr. 10, 1976, Seiten 1849-1857; H.-J. HAPKE et al.: "Zur Pharmakologie von 2'-[2-Hydroxy-3-(propylamino)-propoxy]-3-phenylpropiophenon (Propafenon, SA 79)-hydrochlorid"**

**JOURNAL OF CARDIOVASCULAR PHARMACOLOGY, Band 12, nr. 5, Mai 1988, Seiten 615-619, Raven Press, Ltd, New York, US; D.M. BURNETT et al.: "Propafenone interacts stereoselectivily with beta1- and beta2-adrenergic receptors"**

## Beschreibung

Die Erfindung betrifft die Verwendung der Propafenon-Enantiomeren zur Herstellung von Arzneimitteln zur Behandlung von Arrhythmien bei bestimmten Patientenkollektiven.

Propafenon (INN für 2'-(2-Hydroxy-3-propylaminopropoxy)-3-phenylpropiophenon) mit der Strukturformel

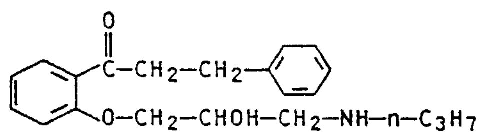

wird in der Form des Hydrochlorids erfolgreich zur Therapie von Herz-Kreislauferkrankungen, insbesondere von Herzrhythmusstörungen eingesetzt.

Propafenon hat neben der antiarrhythmischen Wirkung eine zusätzliche $\beta$-sympatholytische Wirkkomponente.

Propafenon besitzt ein Asymmetriezentrum am Kohlenstoffatom 2 der Aminopropanolseitenkette und wird bisher nur als Racemat verwendet. Das Racemat ist zwar bereits gespalten worden (G. Blaschke u. B. Walther, Liebigs Ann. Chem. 1987, 561-563), jedoch ohne Untersuchung der pharmakologischen Eigenschaften der Enantiomeren.

Diese Untersuchung wurde nun durchgeführt und kam zu überraschenden Ergebnissen mit erheblichen praktischen Konsequenzen, wie weiter unten dargelegt wird.

Die Propafenonenantiomeren können durch stereospezifische Synthese erhalten werden. Hierbei wird das bekannte Phenol I mit einem optisch aktiven $C_3$-Baustein zu einer Zwischenstufe (IV) umgesetzt.

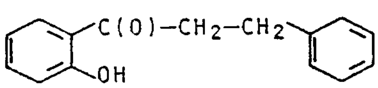

(I)

Als $C_3$-Baustein können beispielsweise Glycidol II oder eines seiner Derivate III eingesetzt werden. In der Formel III

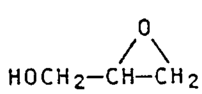

II

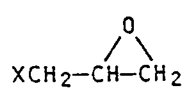

III

bedeutet X eine nukleofuge Abgangsgruppe (die sich durch Nukleophile verdrängen läßt) wie $CF_3SO_3$, $CH_3SO_3$, $CH_3$-$C_6H_4SO_3$ oder Br. Glycidol ist in beiden enantiomeren Formen erhältlich (JOC 51, 3710 (1986), die Derivate III sind nach bekannten Verfahren (JOC 43, 4876 (1978)) daraus herstellbar. Weiterhin können diese $C_3$-Bausteine auch aus Naturstoffen wie Mannitol hergestellt werden (Eur. J. Med. Chem. 17, 69 (1982), TH 42, 447 (1986)).

Die Umsetzung von I mit II oder III wird nach literaturbekannten Verfahren durchgeführt (Heterocycles 20, 1975 (1983); Eur. J. Med. Chem. 17, 69 (1982); JOC 51, 3710 (1986), EP 6615). So kann Glycidol nach den Bedingungen der Mitsunobu-Methode verethert werden, die Verbindungen III werden unter den Bedingungen der Williamson-Synthese umgesetzt. Die Reaktion liefert das optisch aktive Epoxid,

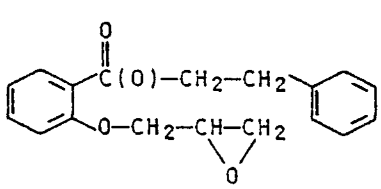

IV,

das in an sich bekannter Weise in (R)- oder (S)-Propafenon übergeführt wird.

Erwartungsgemäß wird die β-blockierende Wirkung von (R,S)-Propafenon vom (S)-Enantiomeren getragen.

Tabelle 1 zeigt, daß die Hemmung der $^3$H-Dihydroalprenololbindung (Herz, Lunge) durch das (S)-Enantiomer signifikant stärker - und durch das (R)-Enantiomer signifikant schwächer ist als durch das (R,S)-Propafenon.

Dagegen unterscheiden sich die Enantiomeren überraschenderweise nicht voneinander hinsichtlich der antiarrhythmischen Wirkung der Klasse I nach Vaughan-Williams (Tabelle 2). Dieser Befund war deshalb nicht zu erwarten, weil normalerweise die therapeutische Wirkung eines Racemats mehr oder weniger weitgehend auf einem Enantiomeren beruht. So ist beispielsweise die Stammverbindung der Klasse I-Antiarrhythmika, das Chinidin, nur in Form des 8(R),9(S)-Enantiomeren wirksam.

Somit liegen mit den Propafenonenantiomeren zwei Verbindungen vor, die aufgrund ihres differenzierten Wirkprofils zur gezielten Therapie von Herzrhythmusstörungen unterschiedlicher Patientenkollektive geeignet sind.

(S)-Propafenon mit stärker β-blockierender Wirkung als Propafenon ist indiziert:

Bei tachykarden Arrhythmieformen, die mit hohen Katecholaminspiegeln einhergehen.

Bei Patienten, die noch nicht unter β-Blocker-Behandlung stehen.

Das (R)-Enantiomer mit schwächer β-blockierender Wirkung als Propafenon ist indiziert:

Bei Patienten, die bereits unter μ-Blocker-Therapie stehen.

Bei älteren (über ca. 50 Jahre alten) und/oder hypotonen und/oder herzinsuffizienten Patienten, bei denen jeweils eine β-Blockade kontraindiziert ist.

Dementsprechend ist Gegenstand der Erfindung die Verwendung eines Propafenon-Enantiomeren zur Herstellung eines Arzneimittels zur Behandlung von Arrhythmien bei den genannten Patientenkollektiven.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise, beispielsweise durch Vermischen des Wirkstoffs mit den an sich in solchen Präparaten üblichen festen und flüssigen Träger- und Hilfsstoffen.

Die Mittel können peroral oder parenteral verabreicht werden. Derartige Zubereitungen sind beispielsweise Tabletten, Filmtabletten, Dragees, Kapseln, Pillen, Pulver, Lösungen oder Suspensionen sowie Infusions- oder Injektionslösungen.

Üblicherweise verwendete pharmazeutisch-technische Hilfsstoffe sind beispielsweise Mannit, Milchzukker, Propylenglykol und Ethanol, Gelatine, Stärke, Talk, Stearinsäure. Polyvinylpyrrolidon. Den Präparaten können gegebenenfalls geschmacksverbessernde Zusätze, Stabilisierungsmittel, Emulgiermittel usw. zugesetzt werden. Voraussetzung ist, daß alle bei der Herstellung der pharmazeutischen Zubereitungen verwendeten Stoffe toxikologisch unbedenklich und mit den verwendeten Wirkstoffen verträglich sind.

Gegebenenfalls werden die erhaltenen erfindungsgemäßen Enantiomeren in das Säureadditionssalz einer physiologisch verträglichen Säure überführt. Eine Zusammenstellung üblicher physiologisch verträglicher Säuren kann aus Fortschritte der Arzneimittelforschung 1966, Birkhäuser-Verlag, Bd. 10, S. 224-285, Deutschland, Schweiz, entnommen werden. Bevorzugt wird Salzsäure.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether, wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der Propefenon-Enantiomeren durch Auflösen der freien Basen in einer wäßrigen Säurelösung hergestellt werden.

Die Wirkstoffgehalte der erfindungsgemäßen pharmazeutischen Präparate liegen im Rahmen des für Propafenonpräparate Üblichen, also bei einer Einzeldosis von 0,1 bis 50, vorzugsweise 0,2 bis 20, insbesondere 1 bis 5 mg pro kg Körpergewicht, für das Hydrochlorid, d.h. für einen Patienten von 70 kg Körpergewicht liegt der Wirkstoffgehalt bei 7 bis 3500, vorzugsweise bei 14 bis 1400, insbesondere bei 70 bis 350 mg.

1. In vitro-Bestimmung der Affinität zu den $\beta_1$- und $\beta_2$-Rezeptorsubtyp mit Hilfe von Kompetitionsversuchen

Hierzu wurden Ansätze mit Rinderherzmembranen (90 % $\beta_1$, 10 % $\beta_2$) oder Rattenlungenmembranen (25 % $\beta_1$, 75 % $\beta_2$) in Tris-HCl (50 mM)/0,1 % Ascorbinsäure (pH 7,4) mit steigenden Konzentrationen von Prüfsubstanz und einer festen Konzentration (1 nM) des Radioliganden $^3$H-Dihydroalprenolol hergestellt. Die unspezifische Bindung wurde mit $10^{-4}$ M Isoproterenol bestimmt.

Nach 60 min Inkubation bei 25°C wurden die Ansätze mit Puffer verdünnt und sofort über Glasfilter (GF/F, Whatman) filtriert und die Menge des auf dem Filter zurückgehaltenen Radioliganden mittels Flüssigkeitsszintillationsmessung bestimmt. Es wurden zwei Versuche in dreifachem Ansatz durchgeführt.

Die Kompetitionskonstanten (Ki-Werte in nM) wurden durch nichtlineare Regressionsanalyse an einem IBM-Rechner in Anlehnung an das Programm "ligand" von Munson und Rodbard (Anal. Biochem. 107, 220, 1980) berechnet.

Tabelle 1

| Hemmung der spezifischen $^3$H-Dihydroalprenolol-Bindung an Rinderherzmembranen (90 % $\beta_1$) und Rattenlungenmembranen (25 % $\beta_1$, 75 % $\beta_2$) | | |
|---|---|---|
| Kompetitionskonstante ($K_i$) mit Vertrauensgrenzen (VG), bestimmt durch simultane Anpassung der Kompetitionskurven | | |
| Substanz | Herz $K_i$ (nM) | Lunge $K_i$ (nM) |
| (R,S)-Propafenon | 74 ( 70 - 77) | 32 ( 31 - 34) |
| (R)-Propafenon | 788 (708 - 868) | 257 (237 - 276) |
| (S)-Propafenon | 59 ( 53 - 64) | 14 ( 13 - 15) |

2. Bestimmung der antiarrhythmischen Wirkung an der Akonitinarrhythmie der Ratte

Als Versuchstiere dienten männliche Sprague-Dawley-Ratten im Gewicht von 180 bis 300 g. Die Narkose erfolgte mit 100 mg/kg Thiobutabarbital i.p. Zur Erzeugung von Arrhythmien wurde Akonitin mit einer Dosierungsgeschwindigkeit von 5 µg/kg • $min^{-1}$ infundiert. Die Prüfsubstanzen wurden intravenös 2 min vor Beginn der Akonitin-Infusion appliziert. Meßgröße ist die Dauer der Akonitin-Infusion, die bei den Tieren im EKG zum Auftreten der ersten Arrhythmien (Verlust von P, ventrikuläre Extrasystolen und Tachykardien) führt. Bei unbehandelten Tieren trat die Akonitin-Arrhythmie nach 3,3 ± 0,11 min auf (n = 120). Aus der linearen Beziehung zwischen log Dosis (mg/kg) der Prüfsubstanzen und der relativen Verlängerung der Akonitin-Infusionsdauer (Δ %) wird die ED50% bestimmt.

Tabelle 2

| Antiarrhythmische Wirkung von (R,S)-Propafenon und seinen Enantiomeren auf Akonitinarrhythmien bei narkotisierten Ratten 5 min nach i.v. Applikation, ED50%; 95 % Vertrauensbereich | |
|---|---|
| Substanz | Antiarrhytmische Wirkung Akonitin-Arrhythmien ED50% mg/kg |
| (R,S)-Propafenon | 0,724 (0,56 - 0,935) |
| (R)-Propafenon | 0,801 (0,44 - 1,46) |
| (S)-Propafenon | 0,676 (0,412 - 1,11) |

Die nachstehenden Beispiele sollen die Erfindung erläutern:

Beispiel 1 (R)-Propafenon • HCl

Zu einer Lösung von 22,6 g (0,1 Mol) 2'-Hydroxy-3-phenylpropiophenon (I), 8,9 g (0,12 Mol) (S)-Glycidol und 31,6 g (0,12 Mol) Triphenylphosphin wurden bei 0 - 5°C 19 ml (0,12 Mol) Azodicarbonsäurediethylester getropft. Man rührte über Nacht bei RT und destillierte das Lösungsmittel ab. Der ölige Rückstand wurde mit 100 ml Propylamin 8 Stdn. unter Rückfluß erhitzt, dann das überschüssige Propylamin abdestilliert. Nach Zugabe von 50 ml 5n HCl wurde 1 Std. auf 50°C erwärmt und filtriert. Beim Abkühlen fiel ein Kristallisat aus, das abgesaugt, mit Ethanol gewaschen und getrocknet wurde. Man erhielt 19,7 g (52 %) (R)-Propafenon • HCl, Fp. 177 - 178°C, $[\alpha]_D^{23}$ = +6,4° (C = 1, $CH_3OH$).

Beispiel 2 (S)-Propafenon • HCl

Zu einer Suspension von 3,7 mMol NaH in 10 ml THF wurden bei 0°C 0,8 g 2′-Hydroxy-3-phenylpropiophenon gegeben. Es entstand eine klare Lösung. Bei -30°C wurden 0,7 g (3,3 mMol) (S)-Trifluormethansulfonsäureglycidylester zugetropft und die Lösung wurde bei -20°C stehen gelassen. Die Mischung wird auf Eis gegossen und mit $CH_2Cl_2$ extrahiert. Die organische Phase wurde getrocknet und das Lösungsmittel abdestilliert. Der ölige Rückstand wurde über Nacht in 5 ml n-Propylamin gerührt. Überschüssiges Amin wurde abdestilliert, der Rückstand in 5 ml Ethanol gelöst und mit etherischer HCl versetzt. Es bildete sich ein farbloses Kristallisat, das abgesaugt und getrocknet wurde. Man erhielt 0,6 g (S)-Propafenon • HCl, Fp. 178 - 179°C,$[\alpha]_D^{23}$ = -6,3° (C = 1, $CH_3OH$).

## Patentansprüche

1. Verwendung allein des (S)-Propafenons zur Herstellung eines Arzneimittels zur Behandlung von Arrhythmien bei Patienten, die nicht bereits unter β-Blocker-Behandlung stehen.

2. Verwendung allein des (S)-Propafenons zur Herstellung eines Mittels zur Bekämpfung von tachykarden Arrhythmien, die mit hohen Katecholaminspiegeln einhergehen.

3. Verwendung allein des (R)-Propafenons zur Herstellung eines Mittels zur Behandlung von Arrhythmien bei Patienten, die bereits unter β-Blocker-Behandlung stehen.

4. Verwendung allein des (R)-Propafenons zur Herstellung eines Mittels zur Behandlung von Arrhythmien bei älteren und/oder hypotonen oder herzinsuffizienten Patienten, bei denen jeweils eine Behandlung mit β-Blockern kontraindiziert ist.

## Claims

1. Use of (S)-propafenone alone for the preparation of a drug for the treatment of arrhythmias in patients who are not being treated with β-blockers.

2. Use of (S)-propafenone alone for the preparation of an agent for the treatment of tachycardiac arrhythmias accompanied by high catecholamine levels.

3. Use of (R)-propafenone alone for the preparation of an agent for the treatment of arrhythmias in patients who are already being treated with β-blockers.

4. Use of (R)-propafenone alone for the preparation of an agent for the treatment of arrhythmias in older patients and/or patients suffering from hypotension or cardiac insufficiency, for whom treatment with β-blockers is contraindicated.

## Revendications

1. Utilisation de la propaphénone-S seule pour préparer un médicament pour le traitement d'arythmies chez des patients qui ne sont pas sous traitement par β-bloquants.

2. Utilisation de la propaphénone-S seule pour préparer un médicament pour la lutte contre des arythmies tachycardiques accompagnées de taux de catécholamine élevés.

3. Utilisation de la propaphénone-R seule pour préparer un médicament pour le traitement d'arythmies chez des patients qui sont déjà traités par des β-bloquants.

4. Utilisation de la propaphénone-R seule pour préparer un médicament pour le traitement d'arythmies chez des patients âgés et/ou hypotoniques ou souffrant d'une insuffisance cardiaque, chez lesquels un traitement avec des $\beta$-bloquants est contre-indiqué.